(19) Europäisches Patentamt
European Patent Office
Office européen des brevets

(11) Veröffentlichungsnummer: **0 411 467 A2**

(12) **EUROPÄISCHE PATENTANMELDUNG**

(21) Anmeldenummer: **90114286.9**

(22) Anmeldetag: **25.07.90**

(51) Int. Cl.5: **C07D 473/34**, //C07F7/10

(30) Priorität: **01.08.89 CH 2849/89**

(43) Veröffentlichungstag der Anmeldung:
**06.02.91 Patentblatt 91/06**

(84) Benannte Vertragsstaaten:
**BE CH DE FR GB IT LI NL**

(71) Anmelder: **LONZA AG**
**Gampel/Wallis Geschäftsleitung Basel**
**CH-4002 Basel(CH)**

(72) Erfinder: **Quittmann, Wilhelm, Dr.**
**Dipl.-Chemiker**
**Rottenstrasse 3**
**Visp, Kanton Wallis(CH)**

(74) Vertreter: **Weinhold, Peter, Dr. et al**
**Patentanwälte Dr. V. Schmied-Kowarzik**
**Dipl.-Ing. G. Dannenberg Dr. P. Weinhold Dr.**
**D. Gudel Dipl.-Ing. S. Schubert Dr. P. Barz**
**Siegfriedstrasse 8**
**D-8000 München 40(DE)**

(54) **Verfahren zur Herstellung von reinem Adenin.**

(57) Reines Adenin wird aus rohem Adenin, das durch 9-Phenyladenin und/oder farbige Beimengungen verunreinigt ist, hergestellt. Dazu wird das Rohprodukt z.B. mit Hexamethyldisilazan in Gegenwart eines Katalysators in N,N′-Bis(trimethylsilyl)adenin übergeführt, welches destilliert und anschliessend durch Hydrolyse in reines Adenin zurückverwandelt wird.

EP 0 411 467 A2

# VERFAHREN ZUR HERSTELLUNG VON REINEM ADENIN

Die vorliegende Erfindung betrifft ein Verfahren zur Herstellung von reinem Adenin, insbesondere aus rohem synthetischem Adenin, welches gegebenenfalls 9-Phenyladenin und/oder weitere Nebenprodukte enthält.

Adenin (6-Aminopurin) spielt in der Natur als Bestandteil der Nucleinsäuren, der Adenosinphosphate und weiterer lebenswichtiger Verbindungen eine bedeutende Rolle. Neben einer direkten pharmazeutischen Anwendung ("Vitamin $B_4$") und der Verwendung in der biochemischen Forschung dient es beispielsweise als Ausgangsmaterial für die Herstellung pharmazeutischer Wirkstoffe (siehe z.B. DE-OS 28 04 723).

Ein wichtiges Verfahren zur Herstellung von Adenin (EP-PS 0 045 503, GB-PS 1 518 784) geht von Malonitril aus, welches mit diazotiertem Anilin zu Phenylazomalonitril gekuppelt wird. Letzteres wird mit Ammoniak und Formamid zu 4,6-Diamino-5-phenylazopyrimidin umgesetzt, welches reduktiv zu 4,5,6-Triaminopyrimidin und Anilin gespalten wird. Aus dem Triaminopyrimidin wird mit Formamid schliesslich Adenin erhalten. Das Verfahren verläuft jedoch nicht ohne Bildung von Nebenprodukten. Als Verunreinigungen enthält das gebildete rohe Adenin typischerweise ca. 1% 9-Phenyladenin und ca. 2 bis 5% braune Substanzen unbekannter Konstitution. Um ein reines Produkt mit einem Gehalt von über 99% zu erhalten, war es bisher erforderlich, das rohe Adenin, gegebenenfalls in Form des Sulfates, mehrmals unter Zusatz von Aktivkohle umzu kristallisieren. Diese Art der Reinigung ist nicht nur sehr umständlich und zeitraubend, sondern verursacht auch erhebliche Substanzverluste, ihre Ausbeute liegt nicht über 75%.

Aufgabe der vorliegenden Erfindung war daher, ein Verfahren zur Verfügung zu stellen, das mit geringem Arbeits-und Zeitaufwand und geringen Substanzverlusten auskommt und gleichzeitig eine hohe Reinheit erzielt.

Erfindungsgemäss wird diese Aufgabe durch ein Verfahren gemäss Patentanspruch 1 gelöst.

Es war bekannt, dass Adenin mit Silylierungsmitteln wie Trimethylchlorsilan in eine destillierbare Bis-(trimethylsilyl)-Verbindung übergeführt werden kann, welche mit Alkohol und/oder Wasser wieder Adenin liefert. Überraschend wurde jedoch gefunden, dass bereits bei einer einfachen Destillation über eine Kolonne geringer Trennleistung (z.B. Vigreux-Kolonne von 30 cm Länge) nicht nur die farbigen Verunreinigungen, sondern auch das (silylierte) 9-Phenyladenin praktisch vollständig abgetrennt werden können.

Die Überführung von Adenin in das Bis(trimethylsilyl)-Derivat wird zweckmässig mit einem der bekannten Silylierungsmittel (siehe z.B. E.P. Plueddemann in: Kirk-Othmer, Encycl.Chem. Technol., 3rd Ed., Vol.20, p.962 (1982)) durchgeführt. Bevorzugt ist jedoch Hexamethyldisilazan, welches im Gegensatz zu dem sonst häufig verwendeten Chlortrimethylsilan keinen Chlorwasserstoff, sondern nur Ammoniak als Nebenprodukt entwickelt. Ammoniak entweicht bei den Reaktionsbedingungen als Gas, während Chlorwasserstoff üblicherweise durch Triethylamin gebunden wird und als Triethylammoniumchlorid durch Fil tration entfernt werden muss. Für die Durchführung des Verfahrens im technischen Massstab ist weiterhin von Vorteil, dass bei der Verwendung von Hexamethyldisilazan keine Korrosionsprobleme auftreten und die Destillation direkt aus dem Reaktionsgefäss der Silylierung erfolgen kann.

Als Katalysatoren für die Silylierung mit Hexamethyldisilazan können die üblichen Katalysatoren (siehe z.B. C.A. Bruynes und T.K. Jurriens, J.Org. Chem. 47 , 3966 (1982)) wie beispielsweise konzentrierte Schwefelsäure, Saccharin oder Imidazol verwendet werden, besonders bevorzugt sind jedoch Polyphosphorsäure oder Polyphosphorsäuretrimethylsilylester. Diese letztgenannten Verbindungen zeichnen sich gegenüber den bisher bekannten Silylierungskatalysatoren durch geringe Korrosivität in Kombination mit vernachlässigbarer Flüchtigkeit aus, so dass sie bei der Destillation vollständig im Rückstand verbleiben. Die bisher bekannten Silylierungskatalysatoren sind nämlich entweder selbst flüchtig oder sie werden wie beispielsweise die Schwefelsäure unter den gegebenen Reaktionsbedingungen selbst silyliert und bilden damit flüchtige Nebenprodukte, die das Destillat verunreinigen.

Die Destillation wird vorzugsweise unter vermindertem Druck in der Grössenordnung von 10 mbar über eine geeignete Kolonne (z.B. Vigreux-Kolonne oder Füllkörperkolonne) vorgenommen, wobei die Vorlage vorteilhaft auf eine Temperatur in der Nähe des Schmelzpunktes (84 bis 87 °C) des Bis(trimethylsilyl)-adenins erwärmt wird, um ein Erstarren des Produktes zu vermeiden.

Die anschliessende Hydrolyse des Destillats kann direkt mit der Schmelze durchgeführt werden, es ist jedoch auch möglich und gegebenenfalls vorteilhaft, das Destillat mit einem geeigneten inerten Lösungsmittel wie beispielsweise Toluol zu verdünnen.

Die Hydrolyse erfolgt vorzugsweise mit heissem Wasser, anschliessend wird vorzugsweise das entstandene Trimethylsilanol, welches gegebenenfalls teilweise oder vollständig zu Hexamethyldisiloxan kondensiert, sowie gegebenenfalls vorhandenes Lösungsmittel mit Wasserdampf abgetrieben (Dampfstrippung). Eine bevorzugte Ausführungsform verwendet einen grossen Überschuss von Wasser, welcher solange

abdestilliert wird, bis kein Silanol bzw. Disiloxan mehr mit übergeht; besonders bevorzugt ist jedoch die Verwendung einer geringeren Wassermenge, wobei das Silanol/Disiloxan und gegebenenfalls das Lösungsmittel durch Einleiten von Wasserdampf abgetrieben wird. Aus dem abdestillierten Gemisch kann das Hexamethyldisiloxan gegebenenfalls durch bekannte Methoden isoliert und anderweitig verwendet werden, es ist auch möglich, daraus wieder nach bekannten Verfahren Trimethylchlorsilan oder Hexamethyldisilazan zurückzugewinnen. Letzteres kann dann gegebenenfalls wieder in der ersten Stufe des erfindungsgemässen Verfahrens eingesetzt werden, so dass sich ein geschlossener Kreislauf des Silylierungsmittels ergibt.

Aus dem Rückstand der Dampfstrippung kann das gereinigte Adenin nach dem Abkühlen durch Zentrifugieren oder Filtration isoliert und auf übliche Weise gewaschen und getrocknet werden.

Die nachfolgenden Beispiele veranschaulichen die Durchführung des erfindungsgemässen Verfahrens. Als Ausgangsmaterial wurden 3 verschiedene Chargen Adenin-Rohprodukt (Muster A, B und C) gemäss Tabelle 1 verwendet.

Die Gehalte an Adenin und 9-Phenyladenin wurden jeweils mittels Hochdruck-Flüssigkeitschromatographie (HPLC) an Nucleosil und UV-Detektor bestimmt.

Die Transparenz wurde jeweils in salzsaurer Lösung (1% in 0,2 n HCl) mit Licht der Wellenlänge 430 nm bei einer Schichtdicke von 1 cm gemessen.

Tabelle 1

| | Gehalt(HPLC, %) | | Transparenz (%) |
|---|---|---|---|
| Muster | Adenin | 9-Phenyladenin | |
| A | 94.5 | 0.2 | 2.1 (nach Filtration) |
| B | 91.5 | 1.1 | ≈0 (intensiv braun) |
| C | 93.9 | 1.4 | 39.5 |

Beispiel 1

In ein 50 l-Rührwerk aus V4A-Stahl wurden 13,5 kg Adenin-Rohware (Muster A), 18,4 kg Hexamethyldisilazan und 10 ml konz. Schwefelsäure bei Raumtemperatur eingefüllt. Das Reaktionsge misch wurde unter Rühren bis zur Beendigung der Ammoniakentwicklung unter Rückfluss gekocht.

Nach beendeter Reaktion wurde das überschüssige Silylierungsmittel bei vermindertem Druck abdestilliert. Das Hauptprodukt N,N′-Bis(trimethylsilyl)adenin wurde über eine kleine Füllkörperkolonne bei 4,5 Torr und einer Siedetemperatur von 162 °C in eine beheizte Vorlage destilliert und anschliessend unter Normaldruck mit 20 l Toluol verdünnt, um eine Kristallisation des Destillates zu vermeiden. Zur Hydrolyse wurden 84,3 kg einer Lösung des silylierten Adenins in 34,6 kg Toluol (aus 2 Ansätzen) in Portionen zu 320 l 75 bis 80 °C heissem Wasser gegeben.

In einer leicht exothermen Reaktion destillierte schon während der Zugabe ein grosser Teil der flüchtigen organischen Komponenten (Toluol, Trimethylsilanol, Hexamethyldisiloxan) ab. Durch äussere Wärmezufuhr wurde weiteres Wasser im Gemisch mit dem Rest dieser flüchtigen Bestandteile abdestilliert. Das Ende dieser Dampfstrippung war erreicht, als eine Probe des Brüdenkondensates vermischt mit dem gleichen Volumen halbkonzentrierter Salzsäure keine Trübung mehr aufwies. Das suspendierte Adenin wurde nach Abkühlen auf Raumtemperatur zentrifugiert, mit Wasser und Methanol gewaschen und im Vakuum getrocknet. Nach dieser Verfahrensweise wurden 24 kg Adenin mit einem Gehalt von über 99,5% erhalten. Weitere analytische Daten können Tabelle 3 entnommen werden.

Beispiel 2

In einem 250 ml Glas-Rundkolben wurden 62,8 g Adenin-Rohware (Muster A) mit 90,0 g Hexamethyldisilazan und 0,462 g Saccharin unter Ausschluss von Luftfeuchtigkeit bis zur Beendigung der Ammoniak-Entwicklung unter Rückfluss erhitzt (8 bis 10 h). Das überschüssige Silylierungsmittel wurde unter Vakuum abdestilliert. Das silylierte Adenin wurde über eine Vigreux-Kolonne (l = 30 cm) bei einem Druck von 7

mbar und einer Kopftemperatur bis 177 °C destilliert. Es wurden so 117 g (95,4%) an N,N′-Bis-(trimethylsilyl)-adenin erhalten. Das Destillat wurde in 90,2 g Toluol gelöst und in 440 ml 75° warmes Wasser eingetragen, und die flüchtigen Produkte der Hydrolyse zusammen mit dem Toluol mit Wasserdampf ausgetrieben. Die wässrige Adenin-Suspension wurde auf Raumtemperatur abgekühlt, filtriert, mit Methanol gewaschen und getrocknet. So wurden 53,47 g (90,2%) reines Adenin erhalten. Die analytischen Daten des Produkts sind ebenfalls in Tabelle 3 aufgeführt.

Beispiele 3 bis 7

Entsprechend dem Beispiel 2 wurden verschiedene Ausgangsmuster mit diversen Silylierungskatalysatoren umgesetzt, wie in der Tabelle 2 aufgezeigt:

Tabelle 2

| Beispiel Nr. | Muster-Bez. | Katalysator | Ausbeute (%) | Bemerkungen |
|---|---|---|---|---|
| 3 | B | Konz. $H_2SO_4$ | 91.9 | |
| 4 | A | Polyphosphorsäuretrimethylsilylester | 85.6 | Destillat als Schmelze ohne Toluol hydrolysiert |
| 5 | A | Polyphosphorsäure | 86.6 | Destillat als Schmelze ohne Toluol hydrolysiert |
| 6 | C | Saccharin | 96.7 | |
| 7 | A | Imidazol | 80.4 | Destillat als Schmelze ohne Toluol hydrolysiert. |

Anmerkung: Als Ausbeute wurde das Verhältnis von erhaltenem reinem Adenin zu der in der Rohware enthaltenen Menge definiert.

Tabelle 3

| Analysenresultate: | | | |
|---|---|---|---|
| Beispiel Nr. | Gehalt (%) | | Transparenz (%) |
| | Adenin | 9-Phenyladenin | |
| 1 | >99.5 | <0.1 | 99.6-99.7 |
| 2 | 99.3 | <0.1 | 98.5 |
| 3 | 99.8 | <0.1 | 100.0 |
| 4 | 99.6 | <0.1 | 97.4 |
| 5 | 99.4 | <0.1 | 98.6 |
| 6 | 99.3 | <0.1 | 98.5 |
| 7 | 99.2 | <0.1 | 98.3 |

## Ansprüche

1. Verfahren zur Herstellung von reinem Adenin aus rohem Adenin, welches gegebenenfalls 9-Phenyladenin und/oder weitere Nebenprodukte enthält, dadurch gekennzeichnet, dass das Adenin in das N,N′-Bis-(trimethylsilyl)-Derivat

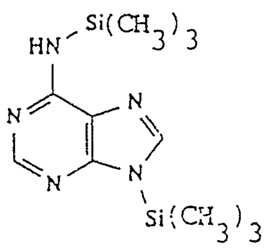

übergeführt, in dieser Form destilliert und anschliessend durch solvolytische Abspaltung der Trimethylsilylgruppen wieder in Adenin zurück umgesetzt wird.

2. Verfahren nach Patentanspruch 1, dadurch gekennzeichnet, dass die Überführung des Adenins in das Bis(trimethylsilyl)-Derivat mit Hexamethyldisilazan erfolgt.

3. Verfahren nach Patentanspruch 2, dadurch gekennzeichnet, dass die Überführung des Adenins in das Bis(trimethylsilyl)-Derivat in Gegenwart von Polyphosphorsäure und/oder Polyphosphorsäuretrimethylsilylester als Katalysator erfolgt.

4. Verfahren nach mindestens einem der Patentansprüche 1 bis 3, dadurch gekennzeichnet, dass die Solvolyse des Bis(trimethylsilyl)adenins mit Wasser erfolgt.

5. Verfahren nach Patentanspruch 4, dadurch gekennzeichnet, dass das bei der Solvolyse des Bis-(trimethylsilyl)adenins entstehende Trimethylsilanol und/oder Hexamethyldisiloxan sowie gegebenenfalls vorhandenes organisches Lösungsmittel durch Einleiten von Wasserdampf abgetrieben werden.